# EUROPÄISCHE PATENTSCHRIFT

(11) **EP 1 461 042 B1**
(45) Veröffentlichungstag und Bekanntmachung des Hinweises auf die Patenterteilung: **13.08.2008**
(21) Anmeldenummer: 02798337.8
(22) Anmeldetag: 14.12.2002
(51) Int. Cl.: A61K 31/519, A61P 25/24

(54) **VERWENDUNG VON DESOXYPEGANIN ZUR BEHANDLUNG DER KLINISCHEN DEPRESSION**
USE OF DESOXYPEGANINE FOR TREATING CLINICAL DEPRESSION
UTILISATION DE DESOXYPEGANINE POUR LE TRAITEMENT DE LA DEPRESSION CLINIQUE

(30) Priorität: 21.12.2001 DE 10163667
(43) Veröffentlichungstag der Anmeldung: 29.09.2004
(73) Patentinhaber: HF ARZNEIMITTELFORSCHUNG GmbH, 59368 Werne (DE)
(72) Erfinder: MOORMANN, Joachim, 59368 Werne (DE); MUCKE, Hermann, A-1160 Wien (AT)
(74) Vertreter: Flaccus, Rolf-Dieter
(86) Internationale Anmeldenummer: PCT/EP2002/014274
(87) Internationale Veröffentlichungsnummer: WO 2003/053445

(56) Entgegenhaltungen:
- WO-A-00/48445
- WO-A-00/48579
- WO-A-00/48582
- WO-A-00/48599
- WO-A-00/48600
- MYCEK M J; HARVEY R A; CHAMPE P C: "Lippincott's Illustrated Reviews: Pharmacology" 1997, LIPPINCOTT-RAVEN , UNITED STATES OF AMERICA * Seite 123, Absatz 4 - Seite 125, Absatz 1 *

## Beschreibung

Die vorliegende Erfindung betrifft die Verwendung von Desoxypeganin zur Herstellung von Arzneimitteln für die Therapie der klinischen Depression, insbesondere Depression in Verbindung mit Demenz oder Alkohol- bzw. Nikotinabusus.

Die unipolare Depression (im Gegensatz zur bipolaren, früher als manisch-depressiv bezeichneten Störung) im Sinne der International Classification of Diseases (ICD-10) bzw. des amerikanischen Diagnostic and Statistics Manual (DSM-IV) ist ein psychisches Zustandsbild, das durch eine Kombination aus gedrückter Stimmung, allgemeinem Verlust von Antrieb und Interesse bei oft gleichzeitiger Ruhelosigkeit, Schlafstörungen, und sozialem Rückzug gekennzeichnet ist. Abgesehen vom Leidensdruck und der Suizidgefährdung der Betroffenen bewirkt die Depression durch Therapiekosten und Arbeitsunfähigkeit einen kaum abschätzbaren volkswirtschaftlichen Gesamtschaden, der z.B. 1990 in den USA etwa 43,7 Milliarden Dollar ausgemacht haben dürfte.

Depression stellt weltweit die bei weitem häufigste psychische Erkrankung dar. Eine Zusammenschau hunderter breit angelegter epidemiologischer Studien ergibt, daß 10 - 25% aller Frauen und 5 - 12% aller Männer zumindest einmal in ihrem Leben an Depression leiden. In den industrialisierten Staaten leiden zu jedem Zeitpunkt etwa 5% der Bevölkerung an Depressionen; hier ist davon auszugehen, daß 15 - 25% aller Patienten, die einen praktischen Arzt oder ein Krankenhaus aufsuchen, an einer Depression leiden. Weltweit trifft dies etwa auf jeden zehnten derartigen Patienten zu. Es handelt sich um eine Erkrankung mit hoher und progressiver Rückfallquote, die zudem in stetiger Zunahme begriffen ist. Die Wahrscheinlichkeit eines Rückfalles steigt von 50% nach einer depressiven Episode auf 70% nach zwei bzw. 90% nach drei solchen Episoden. Die WHO geht davon aus, daß im Jahre 2020 5,7% der Krankheitsbelastung der Weltbevölkerung auf Depression zurückzuführen sein wird, eine Quote, die nur knapp hinter der für kardiovaskuläre Erkrankungen liegt. Aus diesen Informationen lässt sich errechnen, daß zu jedem beliebigen Zeitpunkt 150 Millionen Menschen an klinischer Depression leiden (Mucke HAM.: Next-Generation CNS Therapeutics. Decision Resources, Inc. [Waltham, Mass., USA], 2001).

Es besteht eine erhebliche, in zahlreichen Studien vielfach erwiesene Dreiwegs-Komorbidität zwischen Depression, Alkoholabusus und Nikotinabusus (siehe z.B. J. Hamalainen et al., J. Epidemiol. Community Health 2001; 55(8): 573-576). Traumatische Erlebnisse und chronischer Stress - zwei wesentliche etiologische Faktoren der Depression - sind auch signifikant mit der Ausbildung eines Konsumverhaltes bezüglich Alkohol und Nikotin verbunden (H.J. Little, Alcohol Res. Health 2000; 24(4): 215-224).

Die wirksamsten Arzneimittel zur Therapie der klinischen Depression sind die sogenannten "Trizyklika", trizyklische Verbindungen, die sowohl neuronale Rezeptoren für Serotonin und Norepinephrin blockieren als auch die Wiederaufnahme dieser Neurotransmitter in die jeweiligen Neuronen hemmen und damit deren in der Depression verminderte intrasynaptische Konzentration tendenziell normalisieren. Trizyklika werden auch heute noch breit eingesetzt, obwohl ihre Anwendung mit erheblichen Nebenwirkungen vor allem kardiovaskulärer Art verbunden ist.

Selektive Serotonin-Wiederaufnahmeinhibitoren (SSRI) gelangten etwa ab Mitte der 1980er Jahre zum Einsatz. Sie sind als Klasse betrachtet nicht so stark wirksam wie typische Trizyklika und beginnen erst nach 1-2 Wochen Verzögerung volle Wirkung zu zeigen, jedoch sind ihre Nebenwirkungen erheblich geringer und aufgrund ihrer viel geringeren akuten Toxizität ist Suizid mit ihnen so gut wie unmöglich. Daher wurden SSRI vielfach zu Antidepressiva der ersten Wahl.

Abgesehen von den Nebenwirkungen lassen aber sowohl Trizyklika als auch SSRI erhebliche Lücken in der Therapie der Depression: Etwa 30% aller Patienten sprechen auf keine der beiden Klassen von Antidepressiva in adäquater Weise an (sog. therapierefraktäre Depression). Darüber hinaus gibt es keinen einzelnen Wirkstoff, welcher das Problem des trinkenden depressiven Patienten sowohl auf der Ebene der Depression als auch mittels Reduktion der Alkoholaufnahme behandeln könnte. Für den stark rauchenden depressiven Patienten steht bislang nur ein einziger Wirkstoff (Bupropion, GlaxoSmithKline) zur Verfügung, allerdings nur in Form zweier getrennter und verschieden dosierter Medikamente, von denen eines (Wellbutrin®) lediglich zur Behandlung der Depression und das andere (Zyban®) ausschließlich zur Unterstützung der Rauchentwöhnung zugelassen ist.

Eine Alternative zu den Trizyklika und SSRI stellen die Monoaminoxidase-Inhibitoren (MAOI) dar. Es handelt sich dabei um eine seit ca. 50 Jahren bekannte Klasse von Wirkstoffen, die durch Hemmung des Abbaus aller "Monoamin-Neurotransmitter" (also einschließlich des Dopamins) deren Konzentration im Gehirn erhöht. Die frühen Wirkstoffe dieser Klasse hemmen beide Untertypen der Monoaminoxidase (A und B) in z.T. irreversibler Weise. Aufgrund des Auftretens von Leberschäden einerseits und des "Cheese-Effekts" (einer hypertensiven Krise, ausgelöst durch die Blockierung des Abbaues von mit Nahrungsmitteln wie z.B. Käse aufgenommenen Tyramins) andererseits wurden MAOI zugunsten der Trizyklika aufgegeben.

Es besteht daher nach wie vor erheblicher Bedarf an Antidepressiva, insbesondere solchen, welche sowohl zur Therapie der refraktären Depression als auch in der spezifischen Situation des Alkohol- und/oder Nikotinabusus betreibenden depressiven Patienten besser geeignet sind als handelsübliche Wirkstoffe.

Aufgabe der vorliegenden Erfindung ist daher die Bereitstellung eines Arzneimittels zur Therapie der Demenz, insbesondere der refraktären Demenz, das aber auch in der spezifischen Situation des Alkohol- und/oder Nikotinabusus betreibenden depressiven Patienten besser geeignet ist als handelsübliche Wirkstoffe, das die vorgenannten Nachteile jedoch nicht aufweist.

Desoxypeganin (1,2,3,9-Tetrahydropyrrolo[2,1-b]chinazolin, ein Alkaloid der Summenformel C₁₁H₁₂N₂), kommt in Pflanzen der Familie Zygophyllaceae vor. Die Gewinnung von Desoxypeganin erfolgt vorzugsweise durch Isolierung aus der Steppenraute (*Peganum harmala*) oder durch chemische Synthese. Es ist der pharmazeutischen Wissenschaft aus der Literatur sowie insbesondere durch Patentschriften bekannt.

Die DE-A 199 06 978 bzw. WO 00/48582 beschreibt auf Desoxypeganin basierende Arzneimittel zur Therapie der Drogensucht und Drogenabhängigkeit.

Die DE-A 199 06 975 bzw. WO 00/48599 beschreibt die Verwendung von Desoxypeganin zur Therapie der Alzheimer'schen Demenz.

Die DE-A 199 06 979 bzw. WO 00/48445 beschreibt auf Desoxypeganin basierende Arzneimittel zur Therapie der Nikotinabhängigkeit.

Aufgrund seiner pharmakologischen Eigenschaften wird Desoxypeganin zur Gruppe der reversibel wirkenden Cholinesterasehemmstoffe gezählt. Dass Desoxypeganin nicht nur die Acetylcholinesterase, sondern daneben auch Monoaminoxidasen hemmt, ist aus diesen Veröffentlichungen in allgemeiner Hinsicht bekannt, jedoch wird in diesen Dokumenten zwischen den beiden Subtypen Monoaminoxidase A und B in keiner Weise differenziert. Vor allem wird die Monoaminoxidase-Hemmung durchgängig als eine bloß ergänzende Wirkung beschrieben, welche die als hauptsächlich betrachtete Acetylcholinesterase-Hemmwirkung des Desoxypeganins verstärken soll; ausdrücklich wird z.B. erwähnt, daß der Vorteil der gleichzeitigen Hemmung von Acetylcholinesterase und Monoaminoxidase die auf die Gewichtseinheit bezogen geringere Cholinesterasehemmung (im Vergleich zum prototypischen starken Cholinesterasehemmer Physostigmin) in Hinblick auf die jeweils beanspruchten Anwendungen aufwiegt. Schließlich ist in keiner dieser Schriften Depression als mögliches Anwendungsgebiet angesprochen.

Es wurde nunmehr im Verlauf weiterer pharmakologischer Untersuchungen überraschend festgestellt, daß Desoxypeganin zwar, wie in den oben genannten Schriften beschrieben, die Acetylcholinesterase durchaus hemmt, daß jedoch die quantitative Hauptwirkung in vitro in einer selektiven Hemmung der Monoaminoxidase des Typs A (MAO-A) besteht, wogegen das Enzym des Typs B nicht maßgeblich gehemmt wird. Die bekannten Nebenwirkungen der frühen Monoaminoxidase-Inhibitoren können durch selektive, reversible Inhibitoren der Monoaminoxidase A (RIMA) weitgehend vermieden werden.

Des weiteren wurde - was in Zusammenhang mit dieser doppelten Erkenntnis steht, aber in Bezug auf den Stand der Wissenschaft ebenfalls vollkommen überraschend ist - gefunden, daß Desoxypeganin in einem einschlägigen Tiermodell starke antidepressive und psychomotorisch stimulierende Aktivität zeigt. Dabei tritt die maximale Wirkung bereits bei Dosierungen ein, die in einem Tiermodell der cholinergen Aktivierung noch keinen statistisch signifikanten Effekt zeigt.

Die Hemmwirkung von Desoxypeganin in Bezug auf Monoaminoxidase A aus Rattenhirn (Stamm Wistar) wurde im Konzentrationsbereich von 10 nM bis 10 µM nach der von Medvedev et al. beschriebenen Methode (Biochem Pharmacol 1994; 47(2): 303-308) gemessen und mit Clorgyline als Positivkontrolle verglichen, wobei in beiden Fällen 95 µM [³H]Serotonin in einer Lösung von 1% Dimethylsulfoxid in 20 mM Kaliumdihydrogenphosphat-Puffer pH 7,4 als Substrat diente. Es ergab sich für Desoxypeganin ein Wert von 1,49 µM für die halbmaximale Hemmwirkung (inhibitory concentration 50% = IC₅₀). Dieser Wert liegt fast eine Zehnerpotenz unter dem in einem anderen in vitro - System für die Hemmung der Acetylcholinesterase ermittelten IC₅₀-Wert. Monoaminoxidase B unterlag dagegen bei einer Konzentration von 10 µM Desoxypeganin nur einer Hemmung von 15 - 20%.

Um zu überprüfen, ob diese MAO-A Hemmwirkung in vivo relevant ist, wurde Desoxypeganin im "Forced Swimming Test" (R.D. Porsolt et al., Nature 1977; 266(5604): 730-732) an der Ratte erprobt. Dieses Modell beruht auf dem als "behavioral despair" bezeichneten Verhalten, welches die Tiere in einer für sie bekannten ausweglosen Situation zeigen: Plaziert man sie in ein mit Wasser gefülltes Gefäß, aus dem sie sich nicht befreien können, stellen sie nach einer gewissen Zeit ihre diesbezüglichen Versuche ein und machen nur noch die notwendigsten Schwimmbewegungen. Die Zeit, die die Tiere bis zum Verfall in diese psychomotorische Inaktivierung (die als Surrogat für Depression betrachtet wird) mit Ausbruchsversuchen verbringen, wird gemessen; längere Aktivität entspricht einer antidepressiven Wirkung.

Konkret wurden 50 männliche, etwa 6 Wochen alte Ratten (Stamm Sprague Dawley; bezogen von Charles River UK Ltd.) in 7 Gruppen zu jeweils 10 Tieren geteilt. Am ersten Tag nach Beendigung der Eingewöhnungsphase wurde jedes Tier einzeln 10 Minuten lang in ein zylindrisches Gefäß gesetzt, das mit 25°C warmem Wasser ca. 15 cm tief gefüllt war; dem folgten nach einer Stunde, 19 Stunden und 23 Stunden drei orale Gaben von (je nach Gruppe) entweder Negativkontrolle (Wasser), 15 mg/kg Positivkontrolle (Imipramin·HCl), oder Desoxypeganin·HCl in Dosierungen von 1,0, 2,5, 7,5, 15,0 oder 22,5 mg/kg Körpergewicht. Das mit Hilfe einer Magensonde verabreichte Volumen betrug jeweils 5 ml/kg Körpergewicht. Eine Stunde nach der dritten dieser Behandlungen wurde jedes Tier wiederum für genau 5 Minuten in das Gefäß gebracht, und die in minimaler Mobilität verbrachte Zeit gemessen.

Das starke trizyklische Antidepressivum Imipramin, welches nach den Ergebnissen von Vorversuchen bei der genannten Dosis von 15 mg/kg die maximale in diesem System erzielbare Wirkung definiert, verminderte die in minimaler Mobilität verbrachte Zeit im Vergleich zu Wasser um 56,5% bis 58,9%. Während Desoxypeganin in einer Dosis von 1 mg/kg noch nicht und bei 2,5 mg/kg nur teilweise wirksam war, wurden mit allen Konzentrationen ab 7,5 mg/kg Reduktionen im Bereich von durchschnittlich 41,4% bis 44,1% erzielt. Alle diese Plateau-Werte waren auf dem Niveau p<0,01 statistisch signifikant. Somit blieb der Effekt der Behandlung mit Desoxypeganin zwar bei allen Konzentrationen unter dem in diesem System möglichen Maximum, hatte jedoch schon bei der Hälfte der für die Positivkontrolle verwendeten Dosis (7,5 mg/kg) den Maximalwert für diese Substanz erreicht (siehe Tabelle 1).

Wirkung von Desoxypeganin HCl im Porsolt-Schwimmtest (Ratte, SD-Stamm)

| **Behandlung** | **Dosis (mg/kg p.o.)** | **Erworbene Immobilität (Gruppenmittelwert in Minuten ± Standardabw.)** | **Veränderung relativ zu Kontrollgruppe** |
|---|---|---|---|
| Negativkontrolle (Wasser f. Injektion)) | --- | 3.45±0.60 3.38±0.60 | -- |
| Desoxypeganin hydrochlorid | 1.0 | 3.46±0.83 | +0.3 % |
| Desoxypeganin hydrochlorid | 2.5 | 2.80±1.02 | -18.8 % |
| Desoxypeganin | 7.5 | 1.82**±0.96 | -47.2 % |
| hydrochlorid | | 1.98**±0.83 | -41.4% |
| Desoxypeganin hydrochlorid | 15.0 | 1.90**±0.59 | -44.1% |
| Desoxypeganin hydrochloride | 22.5 | 1.90**±0.54 | -43.8% |
| Positivkontrolle (Imipramin HCl in max. wirksamer Dosis) | 15 | 1.50**±0.49 1.39**±0.52 | -56.5% -58.9% |

| | | | |
|---|---|---|---|
| ** *p*<0.01 | | | |

Da solche Ergebnisse prinzipiell auch durch psychomotorisch aktivierend wirkende Substanzen, die nicht antidepressiv wirken, erzielt werden können, wurde die Wirkung von Desoxypeganin im selben Dosisbereich auch im sogenannten Open Field Paradigma überprüft. Dabei wird die Tatsache ausgenutzt, daß der Aufenthalt in einem offenen, hellen Bereich für die Ratte mit Streß verbunden ist und daher nach Möglichkeit vermieden wird. Ein solches, ebenfalls mit Sprague-Dawley Ratten durchgeführtes Experiment erbrachte jedoch bei einer über 2 Wochen fortgesetzten Behandlung mit täglichen Desoxypeganin-Dosen von 2,5 bis 22,5 mg/kg keinen Hinweis auf psychomotorische Aktivierung, sodaß das Ergebnis des Porsolt-Testes (maximale, mit dem Antidepressivum Imipramin vergleichbare Wirkung bei 7,5 mg/kg p.o.) als aussagekräftig interpretiert werden muß.

Dies ist um so erstaunlicher, als sich in einem weiteren Testsystem, in welchem unter Ausnutzung der Acetylcholinesterase-Hemmwirkung des Desoxypeganins die cholinergen Gedächtnisdefizite von Ratten mit teilweise zerstörten zentralen cholinergen Leitungsbahnen kompensiert wurden, im selben Dosisbereich eine klare lineare Abhängigkeit zeigte, die bei einer oralen Dosis von 7,5 mg/kg statistisch noch nicht signifikant war und bei 22,5 mg/kg ihren Höchstwert noch nicht erreicht hatte. Daraus läßt sich ableiten, daß der im Schwimmtest beobachtbare Effekt bei einem Bruchteil derjenigen Dosierung sein Maximum erreicht, die zur maximalen Aktivierung des cholinergen Systems erforderlich ist, womit nach dem bisherigen Stand der Wissenschaft nicht zu rechnen war.

Somit wirkt Desoxypeganin in einem anerkannten Tiermodell der Depression antidepressiv bzw. psychomotorisch aktivierend, und zwar in maximalem Grad bereits bei einer Dosis, die unter sonst vergleichbaren Bedingungen in einem Verhaltensmodell der cholinergen Kompensation noch absolut suboptimale Wirkung zeigt.

Desoxypeganin ist somit potentiell als Antidepressivum geeignet.

Die Verabreichung von Desoxypeganin kann peroral oder parenteral erfolgen. Für die orale Verabreichung können bekannte Darreichungsformen wie Tabletten, Dragees, Kapseln, Pastillen verwendet werden. Daneben kommen auch flüssige oder halbflüssige Darreichungsformen, beispielsweise als Trinklösungen in Betracht, wobei der Wirkstoff als Lösung oder Suspension vorliegt. Als Löse-oder Suspendierungsmittel können Wasser, wässrige Medien oder pharmakologisch unbedenkliche Öle (vegetabilische oder Mineralöle) verwendet werden.
Vorzugsweise sind die Desoxypeganin enthaltenden Arzneimittel als Depot-Arzneimittel formuliert, welche in der Lage sind, diesen Wirkstoff über einen längeren Zeitraum in kontrollierter Weise an den Organismus abzugeben.

Darüber hinaus kann Desoxypeganin nach der Erfindung auch rektal (beispielweise durch Einführung von Suppositorien), inhalativ (durch Einatmen von Aerosolen mit definierter Konzentration und Größenverteilung der Partikel), transdermal (durch wirkstoffhaltige Pflaster, Einreibelösungen, Gele usw.), transmucosal (im Sinne einer Resorption durch die Mund- und Nasenschleimhaut, wobei der Wirkstoff in der Mundhöhle durch Lösung im Speichel freigesetzt wird, oder durch Sprühlösungen und dergleichen in die Nase eingebracht wird), mittels implantierter Behältnisse (die den Wirkstoff passiv-osmotisch oder gesteuert mittels Minipumpen oder dgl. freisetzen), durch intravenöse, intramuskuläre oder subkutane Injektion und intrazerebroventrikulär verabreicht werden.

In Zusammenhang mit parenteraler Verabreichung können transdermale oder transmucosale Darreichungsformen besonders vorteilhaft für die erfindungsgemäße Verabreichung von Desoxypeganin verwendet werden, insbesondere klebende transdermale therapeutische Systeme (Wirkstoffpflaster), wie sie spezifisch für Desoxypeganin in DE-A 199 06 977 beschrieben sind. Diese ermöglichen es, den Wirkstoff über einen längeren Zeitraum in kontrollierter Weise über die Haut an den zu behandelnden Patienten abzugeben.

Nach der Erfindung kann Desoxypeganin sowohl in Form seiner freien Base als auch als Säureadditionssalz zur Behandlung verwendet werden; als Salze werden Desoxypeganinhydrochlorid und Desoxypeganinhydrobromid bevorzugt. Daneben können auch Salze anderer pharmakologisch akzeptabler Säuren verwendet werden, z. B. Citrat, Tartrat oder Acetat.

Die Arzneiformen, welche gemäß vorliegender Erfindung zur Verabreichung von Desoxypeganin verwendet werden, können einen oder mehrere folgender Zusatzstoffe enthalten:
- Antioxydantien, Synergisten, Stabilisatoren;
- Konservierungsmittel;
- Geschmackskorrigentien;
- Färbemittel;
- Lösemittel, Lösungsvermittler;
- Tenside (Emulgatoren, Solubilisatoren, Benetzer, Entschäumer);
- Viskositäts- und Konsistenzbeeinflusser, Gelbildner;
- Resorptionsbeschleuniger;
- Adsorptionsmittel, Feuchthaltemittel, Gleitmittel;
- Zerfalls- und Lösungsbeeinflusser, Füllmittel (Streckmittel), Peptisatoren;
- Freisetzungsverzögerer.
   Diese Aufzählung ist nicht abschließend; die in Frage kommenden physiologisch unbedenklichen Substanzen sind dem Fachmann bekannt.

Desoxypeganin wird vorzugsweise in einer Arzneizubereitung verabreicht, welche den Wirkstoff in Anteilen von 0,1 bis 90 Gew.-%, besonders bevorzugt in Anteilen von 2 bis 20 Gew.-%, enthält, jeweils berechnet als freies Desoxypeganin. Die erfindungsgemäß verwendeten Desoxypeganin-haltigen Arzneizubereitungen können darüber hinaus die Zusatzstoffe wie Hilfsstoffe, Trägerstoffe und/oder Stabilisatoren in den dem Fachmann bekannten Mengen enthalten.
Die täglich verabreichte Dosis liegt vorzugsweise im Bereich von 0,1 bis 100 mg, insbesondere von 10 bis 50 mg. Sie ist in Abhängigkeit von den individuellen Voraussetzungen entsprechend einzustellen.

## Patentansprüche

1. Verwendung von Desoxypeganin, als freie Base oder als Säureadditionssalz, zur Herstellung eines Arzneimittels zur Behandlung der klinischen Depression.

2. Verwendung nach Anspruch 1, **dadurch gekennzeichnet, daß** es sich bei der klinischen Depression um eine therapierefraktäre Depression handelt.

3. Verwendung nach Anspruch 1 oder 2, **dadurch gekennzeichnet, daß** es sich bei der Depression um eine Depression in Verbindung mit Demenz handelt.

4. Verwendung nach einem der Ansprüche 1 bis 3, **dadurch gekennzeichnet**, es sich bei der Depression um eine Depression in Verbindung mit Suchtmittel- oder Rauschmittelmißbrauch handelt.

5. Verwendung nach einem der Ansprüche 1 bis 4, **dadurch gekennzeichnet, daß** es sich bei dem Suchtmittelmißbrauch um Alkohol- und/oder Nikotinabusus handelt.

6. Verwendung nach einem der Ansprüche 1 bis 5, **dadurch gekennzeichnet, daß** das Arzneimittel den Wirkstoff Desoxypeganin in Anteilen von 0,1 bis 90 Gew.-%, vorzugsweise 2 bis 20 Gew.-%, berechnet als freies Desoxypeganin, enthält.

7. Verwendung nach einem der Ansprüche 1 bis 6, **dadurch gekennzeichnet, daß** das Arzneimittel eine Depotwirkung aufweist.

8. Verwendung nach einem der Ansprüche 1 bis 7, **dadurch gekennzeichnet, daß** das Arzneimittel ein oral verabreichbares Arzneimittel ist.

9. Verwendung einem der Ansprüche 1 bis 7, **dadurch gekennzeichnet, daß** das Arzneimittel ein parenteral verabreichbares Arzneimittel ist.

10. Verwendung nach Anspruch 9, **dadurch gekennzeichnet, daß** das Arzneimittel ein transdermal verabreichbares Arzneimittel ist.

## Claims

1. Use of deoxypeganine, as a free base or as an acid addition salt, for the production of a drug for treating clinical depression.

2. Use according to claim 1, **characterized in that** said clinical depression is a therapy-refractory depression.

3. Use according to claim 1 or 2, **characterized in that** said depression is a depression in connection with dementia.

4. Use according to any one of claims 1 to 3, **characterized in that** said depression is a depression in connection with abuse of addictive substances or narcotics.

5. Use according to any one of claims 1 to 4, **characterized in that** said addictive substance abuse is alcohol and/or nicotine abuse.

6. Use according to any one of claims 1 to 5, **characterized in that** the said drug contains the active agent deoxypeganine in proportions of 0.1 to 90%-wt, preferably 2 to 20%-wt, calculated as free deoxypeganine.

7. Use according to any one of claims 1 to 6, **characterized in that** the said drug has depot action.

8. Use according to any one of claims 1 to 7, **characterized in that** said drug is an orally administrable drug.

9. Use according to any one of claims 1 to 7, **characterized in that** said drug is a parenterally administrable drug.

10. Use according to claim 9, **characterized in that** said drug is a transdermally administrable drug.

## Revendications

1. Utilisation de désoxypéganine, sous la forme d'une base libre ou sous la forme d'un sel d'addition d'acide, pour la préparation d'un médicament destiné au traitement de la dépression clinique.

2. Utilisation selon la revendication 1, **caractérisée en ce qu'**il s'agit, en ce qui concerne la dépression clinique, d'une dépression réfractaire aux thérapies.

3. Utilisation selon la revendication 1 ou 2, **caractérisée en ce que** qu'il s'agit, en ce qui concerne la dépression, d'une dépression en liaison avec une démence.

4. Utilisation selon l'une quelconque des revendications 1 à 3, **caractérisée en ce que**, en ce qui concerne la dépression, il s'agit d'une dépression en liaison avec un abus de drogues ou de stupéfiants.

5. Utilisation selon l'une quelconque des revendications 1 à 4, **caractérisée en ce que**, en ce qui concerne l'abus de drogues, il s'agit d'abus d'alcool et/ou d'abus de nicotine.

6. Utilisation selon l'une quelconque des revendications 1 à 5, **caractérisée en ce que** le médicament contient la substance active désoxypéganine dans des fractions de 0,1 à 90 % en poids, de préférence de 2 à 20 % en poids, calculés comme désoxypéganine libre.

7. Utilisation selon l'une quelconque des revendications 1 à 6, **caractérisée en ce que** le médicament présente un effet de libération prolongée.

8. Utilisation selon l'une quelconque des revendications 1 à 7, **caractérisée en ce que** le médicament est un médicament qui peut être administré par voie orale.

9. Utilisation selon l'une quelconque des revendications 1 à 7, **caractérisée en ce que** le médicament est un médicament qui peut être administré par voie parentérale.

10. Utilisation selon la revendication 9, **caractérisée en ce que** le médicament est un médicament qui peut être administré par voie transdermique.
